(19) Europäisches Patentamt
European Patent Office
Office européen des brevets

(11) Veröffentlichungsnummer: **0 012 225 B1**

(12) **EUROPÄISCHE PATENTSCHRIFT**

(45) Veröffentlichungstag der Patentschrift: **28.07.82**

(51) Int. Cl.³: **C 07 C 47/45,** C 07 C 47/225, C 07 C 45/50, C 11 B 9/00

(21) Anmeldenummer: **79104454.8**

(22) Anmeldetag: **12.11.79**

(54) **Neue Aldehyde, deren Herstellung und deren Verwendung als Riechstoffe.**

(30) Priorität: **16.11.78 DE 2849720**

(43) Veröffentlichungstag der Anmeldung: **25.06.80 Patentblatt 80/13**

(45) Bekanntmachung des Hinweises auf die Patenterteilung: **28.07.82 Patentblatt 82/30**

(84) Benannte Vertragsstaaten: **BE CH DE FR GB IT SE**

(56) Entgegenhaltungen:
**GB-A-1 228 201**
**US-A-3 965 192**

(73) Patentinhaber: **Henkel Kommanditgesellschaft auf Aktien, Postfach 1100 Henkelstrasse 67, D-4000 Düsseldorf 1 (DE)**

(72) Erfinder: **Hagen, Jens, Dr., Dieselstrasse 5, D-6834 Ketsch (DE)**
Erfinder: **Bruns, Klaus, Dr., Notburgaweg 6, D-4150 Krefeld-Traar (DE)**

**EP 0 012 225 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Aldehyde, deren Herstellung und deren Verwendung als Riechstoffe

Es wurde gefunden, dass durch Hydroformylierung von Caryophyllen erhaltene Aldehydgemische wertvolle neue Riechstoffe mit Guajak- und Holz-Note sowie aussergewöhnlicher Haftfestigkeit darstellen.

Die Herstellung der neuen Verbindungsgemische erfolgt zweckmässigerweise durch Umsetzung von Caryophyllen mit Kohlenmonoxid und Wasserstoff bei 70-160°C und unter einem Druck von 100-250 bar. Als Katalysatoren werden dabei Gemische aus tertiären Phosphinen und diese tertiären Phosphine enthaltenden Rhodiumcarbonylkomplexen verwendet. Als tertiäre Phosphine eignen sich Trialkylphosphine, deren Alkylreste 1 bis 20 Kohlenstoffatome aufweisen, sowie Triphenylphosphine, deren Phenylreste durch Alkyl- oder Alkoxygruppen mit 1 bis 4 Kohlenstoffatomen substituiert sein können, insbesondere jedoch Triphenylphosphin. In den Katalysatormischungen liegt die Molzahl von insgesamt vorhandenem Phosphin pro Grammatom Rhodium im Bereich von 20 bis 200.

Die genaue Zusammensetzung der katalytisch wirksamen Rhodiumcarbonylkomplexe ist nicht bekannt. Man kann jedoch davon ausgehen, dass es sich um Rhodiumkomplexe handelt, in denen ein oder mehrere Carbonylliganden durch Phosphinliganden ersetzt sind. Die tatsächlich wirksame Komplexverbindung wird in jedem Fall in situ unter den Bedingungen der Hydroformylierung gebildet. Die hierzu erforderliche Menge Rhodium kann deshalb dem Reaktionsgemisch in Form von Rhodiumchlorid, Rhodiumoxid, Rhodiumsalzen von Fettsäuren, Rhodiumchelaten, Rhodiumcarbonyl oder dimeren Rhodiumcarbonylchlorid zugeführt werden. Vorzugsweise werden Rhodiumkomplexe eingesetzt, die das im Katalysatorgemisch vorhandene Phosphin bereits als Ligand enthalten, insbesondere die Verbindung $RhCl(CO)[P(C_6H_5)_3]_2$.

Die Rhodiumverbindungen setzt man vorteilhaft in solchen Mengen ein, dass, bezogen auf Caryophillen, 5 bis 5 000 ppm, vorzugsweise 15 bis 400 ppm, berechnet als Metall, vorhanden sind.

Die Umsetzung kann in Abwesenheit von Lösungsmitteln durchgeführt werden. Es hat sich jedoch als zweckmässig erwiesen, Lösungsmittel zu verwenden, wobei u.a. gesättigte Kohlenwasserstoffe wie Pentan, Hexan, Heptan, Cyclohexan, Benzol, Toluol, oder Xylol, Ether wie Tetrahydrofuran und Dioxan, Alkohole wie Methanol, Ethanol und Isopropanol, oder Diole wie Ethylenglykol und Propylenglykol in Frage kommen. Bevorzugt wird die Hydroformylierung in gesättigten Kohlenwasserstoffen oder Ethern durchgeführt.

Die Aufarbeitung des Reaktionsgemisches erfolgt auf dem Wege der Destillation, die zweckmässigerweise in einer Inertgasatmosphäre, z.B. in einer Stickstoffatmosphäre durchgeführt wird.

Das aus der beschriebenen Hydroformylierung von Caryophyllen resultierende Reaktionsprodukt stellt nach dem Ergebnis der gaschromatischen Untersuchung ein Gemisch aus vier Stoffen dar. Auf Grund der IR-spektroskopischen Daten wird angenommen, dass es sich um die jeweils möglichen Stereoisomeren der Verbindungen I und II handelt,

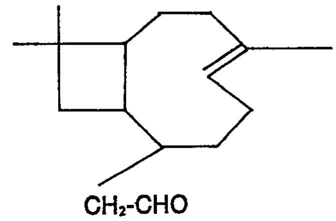

I

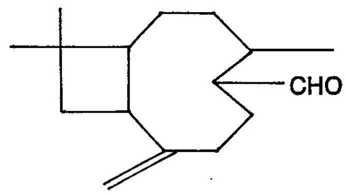

II

wobei die beiden Species der Formel II in erheblich geringerer Menge vorhanden sind als die der Formel I.

Das Gemisch hat Riechstoffeigenschaften und kann mit anderen Riechstoffen in verschiedenen Mengenverhältnissen zu neuen Riechstoffkompositionen gemischt werden. Im allgemeinen wird sich ihr Anteil in den Riechstoffkompositionen in den Mengen von 1 bis 50 Gewichtsprozent, bezogen auf die gesamte Komposition, bewegen. Derartige Kompositionen können direkt als Parfüm oder auch zur Parfümierung von Kosmetika, wie Cremes, Lotionen, Duftwässern, Aerosolen, Toiletteseifen usw. dienen. Sie können aber auch zur Geruchsverbesserung von technischen Produkten wie Wasch- und Reinigungsmitteln, Weichspülern, Textilbehandlungsmitteln eingesetzt werden. Zur Parfümierung der verschiedenen Produkte werden diesen die Kompositionen im allgemeinen in Konzentrationen von 0,05 bis 2 Gewichtsprozent zugesetzt.

Beispiel

In einem 1-l-Hubrührautoklaven aus rostfreiem Stahl wurden 130 g (0,63 Mol) Caryophyllen, 1,9 g (7,25 mmol) Triphenylphosphin und 0,1 g (0,145 mmol) $RhCl(CO)[P(C_6H_5)_3]_2$ und 150 ml Tetrahydrofuran miteinander vermischt. Der Autoklav wurde mit Synthesegas gespült. Anschliessend wurde ein aus gleichen Volumina Wasserstoff und Kohlenmonoxid bestehendes Gemisch bis zu einem Druck von 120 bar aufgepresst. Der Autoklaveninhalt wurde unter Rühren auf 120°C aufheizt, anschliessend 6$^1/_2$ Stunden lang auf 120 bis 130°C gehalten, dann auf Raumtemperatur abgekühlt. Das erhaltene Reaktionsgemisch wurde unter Stickstoffatmosphäre fraktioniert destilliert. Nach dem Abtrennen des Tetrahydrofurans gingen bei 95-110°C/

0,13 mbar 88 g Aldehydgemisch (60% d.Th.) über.

Die gaschromatographische Untersuchung zeigte, dass das Produkt ein Vierkomponentengemisch darstellt.

Carbonylzahl: 110 (theor. 119,7)

Das Produkt zeigte folgendes IR-Spektrum (Film):

3 070 $cm^{-1}$; 2 705 $cm^{-1}$; 1 725 $cm^{-1}$ (CHO); 1 635 $cm^{1}$ (C=C 25 exocyclisch); 1 380 $cm^{-1}$ (gem. di-Methyl); 1 364 $cm^{-1}$; 860 $cm^{-1}$ (C=C trisubstituiert).

Geruch: Guajak-Note, Holz-Note.

**Patentansprüche**

1. Aldehydgemische, dadurch erhältlich, dass man Caryophyllen bei 70 bis 160°C unter einem Druck von 100 bis 250 bar und in Gegenwart eines Katalysatorgemisches aus tertiären Phosphinen und diese tertiären Phosphine enthaltenden Rhodiumcarbonylkomplexen mit Wasserstoff und Kohlenmonoxid umsetzt.

2. Verfahren zur Herstellung der Aldehydgemische nach Anspruch 1, dadurch gekennzeichnet, dass man Caryophyllen bei 70 bis 160°C, unter einem Druck von 100 bis 250 bar und in Gegenwart eines Katalysatorgemisches aus tertiären Phosphinen und diese tertiären Phosphine enthaltenden Rhodiumcarbonylkomplexen mit Wasserstoff und Kohlenmonoxid umsetzt.

3. Verfahren nach Anspruch 2, dadurch gekennzeichnet, dass das Katalysatorgemisch Trialkylphosphine, deren Alkylreste 1 bis 20 Kohlenstoffatome aufweisen, enthält.

4. Verfahren nach Anspruch 2, dadurch gekennzeichnet, dass das Katalysatorgemisch Triphenylphosphin oder Triphenylphosphine, deren Phenylreste durch Alkyl- oder Alkoxygruppen mit 1 bis 4 Kohlenstoffatomen substituiert sind, enthält.

5. Verfahren nach den Ansprüchen 2 bis 4, dadurch gekennzeichnet, dass in dem Katalysatorgemisch die Molzahl des insgesamt vorhandenen Phosphins pro Grammatom Rhodium im Bereich von 20 bis 200 liegt.

6. Verfahren nach den Ansprüchen 2, 4 und 5, dadurch gekennzeichnet, dass das Katalysatorgemisch aus Triphenylphosphin und $RhCl(CO[P(C_6H_5)_3]_2$ gebildet wird.

7. Verwendung der Aldehydgemische nach Anspruch 1 als Riechstoffe.

**Claims**

1. Aldehyde mixtures obtainable by reacting caryophyllin with hydrogen and carbon monoxide at 70 to 160°C under a pressure of from 100 to 250 bars and in the presence of a catalyst mixture of tertiary phosphines and rhodium carbonyl complexes containing these tertiary phosphines.

2. A process for producing the aldehyde mixtures claimed in Claim 1, characterised in that caryophillin is reacted with hydrogen and carbon monoxide at 70 to 160°C under a pressure of from 100 to 250 bars and in the presence of a catalyst mixture of tertiary phosphines and rhodium carbonyl complexes containing these tertiary phosphines.

3. A process as claimed in Claim 2, characterised in that the catalyst mixture contains trialkyl phosphines of which the alkyl radicals contain from 1 to 20 carbon atoms.

4. A process as claimed in Claim 2, characterised in that the catalyst mixture contains triphenyl phosphine or triphenyl phosphines of which the phenyl radicals are substituted by alkyl or alkoxy groups containing from 1 to 4 carbon atoms.

5. A process as claimed in Claims 2 to 4, characterised in that, in the catalyst mixture, the molar number of the phosphine present as a whole per gram atom of rhodium is in the range from 20 to 200.

6. A process as claimed in Claims 2, 4 and 5, characterised in that the catalyst mixture consists of triphenyl phosphine and $RhCl(CO[P(C_6H_5)_3]_2$.

7. The use of the aldehyde mixtures claimed in Claim 1 as fragrances.

**Revendications**

1. Mélanges d'aldéhydes pouvant être obtenus par réaction du caryophyllène avec l'hydrogène et l'oxyde de carbone, à une température de 70 à 160°C, sous une pression de 100 à 250 bars et en présence d'un mélange de catalyseurs consistant en phosphines tertiaires et complexes de rhodium-carbonyle contenant ces phosphines tertiaires.

2. Procédé de préparation des mélanges d'aldéhydes selon la revendication 1, caractérisé en ce que l'on fait réagir le caryophyllène avec l'hydrogène et l'oxyde de carbone à des températures de 70 à 160°C, sous une pression de 100 à 250 bars et en présence d'un mélange de catalyseurs consistant en phosphines tertiaires et complexes de rhodium-carbonyle contenant ces phosphines tertiaires.

3. Procédé selon la revendication 2, caractérisé en ce que le mélange de catalyseurs contient des trialkylphosphines dont les groupes alkyles contiennent de 1 à 20 atomes de carbone.

4. Procédé selon la revendication 2, caractérisé en ce que le mélange de catalyseurs contient de la triphénylphosphine ou des triphénylphosphines dont les groupes phényles sont substitués par des groupes alkyles ou alcoxy en $C_1$-$C_4$.

5. Procédé selon les revendications 2 à 4, caractérisé en ce que, dans le mélange de catalyseurs, le nombre de moles de phosphine présentes au total par atome-gramme de rhodium se situe dans l'intervalle de 20 à 200.

6. Procédé selon les revendications 2, 4 et 5, caractérisé en ce que le mélange de catalyseurs est constitué de triphénylphosphine et de $RhCl(CO[P(C_6H_5)_3]_2$.

7. Utilisation des mélanges d'aldehydes selon la revendication 1 en tant que matières aromatiques.